# EUROPEAN PATENT SPECIFICATION

(11) **EP 4 192 326 B1**
(45) Date of publication and mention of the grant of the patent: **30.10.2024**
(21) Application number: 21791180.9
(22) Date of filing: 21.09.2021
(51) Int. Cl.: A61B 1/00, A61B 1/015, B05B 7/24, B05B 9/04

(54) **UNIVERSAL FLUID CONTAINER ENCLOSURE SYSTEMS**
VERSCHLUSS-SYSTEM ZUM VERSCHLIESSEN EINES FLUIDBEHÄLTERS
SYSTÈMES DE FERMETURE DE CONTENEURS DE FLUIDE À FILETAGE UNIVERSEL

(30) Priority: 28.09.2020 US 202063084274 P; 28.09.2020 US 202063084284 P; 28.09.2020 US 202063084292 P; 28.09.2020 US 202063084297 P
(43) Date of publication of application: 14.06.2023
(73) Proprietor: Boston Scientific Scimed, Inc., Maple Grove, MN 55311 (US)
(72) Inventor: CHRISTAKIS, Laura E., Maple Grove, Minnesota 55311 (US); WALES, Ryan V., Maple Grove, Minnesota 55311 (US); BRECHBIEL, Scott E., Maple Grove, Minnesota 55311 (US)
(74) Representative: Vossius & Partner Patentanwälte Rechtsanwälte mbB
(86) International application number: PCT/US2021/051309
(87) International publication number: WO 2022/066641

(56) References cited:
- WO-A1-2013/061319
- US-A1- 2006 111 611
- US-A1- 2006 149 131
- US-A1- 2007 112 336
- US-A1- 2019 117 046

## Description

### FIELD

The present disclosure relates generally to enclosures for fluid containers. In particular, the present disclosure relates to universal fluid container enclosures for endoscopic systems.

### BACKGROUND

Endoscopy procedures that use typical endoscopes for both therapeutic and diagnostic cases usually have some common functionalities available to an operator. One of the common functionalities includes the ability to insufflate a patient by passing a fluid, such as air or carbon dioxide, through the endoscope in a controlled manner into a target luminal space. Another of the common functionalities includes the ability to flush water across the imaging lens to clear the field of view. Yet another of the common functionalities includes the ability to irrigate the lumen to clean surfaces and aid in flushing/suctioning debris during a procedure. Oftentimes, these common functionalities, among others, are facilitated by one or more fluid containers and/or fluid sources. For example, an air pump or carbon dioxide source for insufflation, a water bottle for lens cleaning, and/or a sterile water bottle for irrigation. In some cases, a hybrid tubing set may be used for both lens cleaning and irrigation from a sterile water bottle. The one or more fluid containers and/or sources, each potentially with a different size and/or configuration, must be attached to the tubing set of the endoscope.

US 2006/149131 A1 discloses a sheathing assembly and endoscopic tool combination for endoscope, having a proximal end of the sleeve engaging shaft of the tool so that as the tool is withdrawn the sleeve unfurls from dispenser and covers the shaft of the tool.

It is with all of the above considerations in mind that the improvements of the present disclosure may be useful.

### SUMMARY

This summary of the disclosure is given to aid understanding, and one of skill in the art will understand that each of the various features of the disclosure may advantageously be used separately in some instances, or in combination with other features of the disclosure in other instances. No limitation as to the scope of the claimed subject matter is intended by either the inclusion or non-inclusion of elements, components, or the like in this summary.

In one embodiment, the present disclosure relates to an enclosure system for a fluid container. For example, an enclosure system for enclosing a fluid container may include a plug having an outer diameter larger than an opening of a neck of the fluid container, a first plug end and a second plug end may be arranged on opposing sides of the plug, the plug may be configured to deform responsive to pressure to reduce the outer diameter to fit the first plug end through the opening and into the neck of the fluid container, and a sleeve forming a hollow flexible cylinder extending from the second plug end, the sleeve having a first sleeve end coupled to the second plug and a second sleeve end arranged opposite the first sleeve end, the first sleeve end may be configured to fold at least a portion of the sleeve outward and down toward the second plug end to arrange at least a portion of the sleeve over an outer neck surface of the neck when the first plug end is installed within the neck.

In some embodiments of the enclosure system, the first plug end may be configured to expand to engage an inner surface of the neck to form a seal responsive to removal of the pressure.

In various embodiments of the enclosure system, an outer sleeve surface of the sleeve may be configured to engage the outer neck surface when the sleeve is folded over the outer neck surface to form a seal between the sleeve and the neck.

In some embodiments of the enclosure system, the at least one opening may be formed in the second plug end for at least one tube of a tube set to extend through the plug to access a fluid arranged with the fluid container.

In various embodiments of the enclosure system, the enclosure system may include at least one liner arranged within the at least one opening to maintain a shape of opening when the plug is deformed.

In exemplary embodiments of the enclosure system, the enclosure system may include a plunger having a plunger rod extending through the sleeve and the plug, the plunger rod may include a first plunger end anchored to the first plug end and a second plunger end arranged opposite the first plunger end, the second plunger end may include a plunger handle.

In various embodiments of the enclosure system, the plunger handle may be configured to move in a direction toward the first plug end responsive to a force to cause the first plunger end to pull on the first plug end to stretch the plug and reduce the outer diameter to fit the first end in the opening of the neck.

In some embodiments of the enclosure system, the first plug end may be configured to expand to engage an inner surface of the neck to form a seal responsive to removal of the force.

In various embodiments of the enclosure system, the plunger rod may be removably coupled to the anchor.

In one embodiment, the present disclosure relates to an enclosure system for a fluid container. For example, an enclosure system for enclosing a fluid container may include a cap comprising at least one protrusion extending from an internal side of a base portion of the cap, the at least one protrusion may be configured to engage an outer neck surface of an opening of the neck of the fluid container as the cap is being pressed down onto the neck to cause the base portion to expand to allow the at least one protrusion to engage at least one thread on the outer surface of neck, the base portion may be biased toward the neck to hold the cap on the neck, and a sealing material may be arranged within a top internal portion of the cap, the sealing material may be configured to contact at least a portion of the outer neck surface to form a seal between the enclosure system and the neck.

In some embodiments of the enclosure system, the sealing material may be formed of a malleable material comprising at least one of a polymer, silicone, a wax, or a combination thereof.

In various embodiments of the enclosure system, the at least one protrusion may be or may include at least one hook.

In exemplary embodiments of the enclosure system, the at least one protrusion may be or may include at least one ridge extending circumferentially around at least a portion of the internal surface.

In some embodiments of the enclosure system, the cap may be configured to withstand a pressure of about 8 psi when installed on the fluid container.

In various embodiments of the enclosure system, at least one opening may be formed in the cap for at least one tube of a tube set to extend through the cap to access a fluid arranged with the fluid container.

In one embodiment, the present disclosure relates to an enclosure system for a fluid container. For example, an enclosure system may include a plug having an outer diameter larger than an opening of a neck of the fluid container, a first plug end and a second plug end arranged on opposing sides of the plug, the plug may be configured to deform responsive to pressure to reduce the outer diameter to fit the first plug end through the opening and into the neck of the fluid container, and a sleeve forming a hollow flexible cylinder extending from the second plug end, the sleeve having a first sleeve end coupled to the second plug and a second sleeve end arranged opposite the first sleeve end, the first sleeve end configured to fold at least a portion of the sleeve outward and down toward the second plug end to arrange at least a portion of the sleeve over an outer neck surface of the neck when the first plug end is installed within the neck.

In some embodiments of the enclosure system, the first plug end may be configured to expand to engage an inner surface of the neck to form a seal responsive to removal of the pressure.

In various embodiments of the enclosure system, an outer sleeve surface of the sleeve may be configured to engage the outer neck surface when the sleeve is folded over the outer neck surface to form a seal between the sleeve and the neck.

In some embodiments of the enclosure system, at least one opening may be formed in the second plug end for at least one tube of a tube set to extend through the plug to access a fluid arranged with the fluid container.

In various embodiments of the enclosure system, the enclosure system may include at least one liner arranged within the at least one opening to maintain a shape of opening when the plug is deformed.

In some embodiments of the enclosure system, the enclosure system may include a plunger having a plunger rod extending through the sleeve and the plug, the plunger rod having a first plunger end anchored to the first plug end and a second plunger end arranged opposite the first plunger end, the second plunger end having a plunger handle.

In exemplary embodiments of the enclosure system, the plunger handle may be configured to move in a direction toward the first plug end responsive to a force to cause the first plunger end to pull on the first plug end to stretch the plug and reduce the outer diameter to fit the first end in the opening of the neck.

In various embodiments of the enclosure system, the first plug end may be configured to expand to engage an inner surface of the neck to form a seal responsive to removal of the force.

In some embodiments of the enclosure system, the plunger rod may be removably coupled to the anchor.

In one embodiment, the present disclosure relates to an enclosure system for a fluid container. For example, an enclosure system for enclosing a fluid container may include a cap comprising at least one protrusion extending from an internal side of a base portion of the cap, the at least one protrusion may be configured to engage an outer neck surface of an opening of the neck of the fluid container as the cap is being pressed down onto the neck to cause the base portion to expand to allow the at least one protrusion to engage at least one thread on the outer surface of neck, the base portion may be biased toward the neck to hold the cap on the neck, and a sealing material may be arranged within a top internal portion of the cap, the sealing material may be configured to contact at least a portion of the outer neck surface to form a seal between the enclosure system and the neck.

In some embodiments of the enclosure system, the sealing material may be formed of a malleable material comprising at least one of a polymer, silicone, a wax, or a combination thereof.

In various embodiments of the enclosure system, the at least one protrusion may be or may include at least one hook.

In exemplary embodiments of the enclosure system, the at least one protrusion may be or may include at least one ridge extending circumferentially around at least a portion of the internal surface.

In various embodiments of the enclosure system, the cap may be configured to withstand a pressure of about 8 psi when installed on the fluid container.

In some embodiments of the enclosure system, at least one opening may be formed in the cap for at least one tube of a tube set to extend through the cap to access a fluid arranged with the fluid container.

In one embodiment, the present disclosure relates to an apparatus. For example, an apparatus may include a fluid container, a tube set arranged and configured to access fluid within the fluid container, the tube set coupled to an endoscope for use during an endoscopic procedure, an enclosure system configured to enclose the fluid container, the enclosure system configured to enclose fluid containers having an opening of about 1 cm to about 10 cm.

In some embodiments of the apparatus, the enclosure system may include a plug having an outer diameter larger than an opening of a neck of the fluid container, a first plug end and a second plug end arranged on opposing sides of the plug, the plug configured to deform responsive to pressure to reduce the outer diameter to fit the first plug end through the opening and into the neck of the fluid container, and a sleeve forming a hollow flexible cylinder extending from the second plug end, the sleeve having a first sleeve end coupled to the second plug and a second sleeve end arranged opposite the first sleeve end, the first sleeve end configured to fold at least a portion of the sleeve outward and down toward the second plug end to arrange at least a portion of the sleeve over an outer neck surface of the neck when the first plug end is installed within the neck.

In various embodiments of the apparatus, the enclosure system may include a plunger having a plunger rod extending through the sleeve and the plug, the plunger rod having a first plunger end anchored to the first plug end and a second plunger end arranged opposite the first plunger end, the second plunger end having a plunger handle.

In exemplary embodiments of the apparatus, the enclosure system may include a cap comprising at least one protrusion extending from an internal side of a base portion of the cap, the at least one protrusion configured to engage an outer neck surface of an opening of the neck of the fluid container as the cap is being pressed down onto the neck to cause the base portion to expand to allow the at least one protrusion to engage at least one thread on the outer surface of neck, the base portion biased toward the neck to hold the cap on the neck, and a sealing material arranged within a top internal portion of the cap, the sealing material to contact at least a portion of the outer neck surface to form a seal between the enclosure system and the neck.

In some embodiments of the apparatus, the sealing material may be formed of a malleable material comprising at least one of a polymer, silicone, a wax, or a combination thereof.

### BRIEF DESCRIPTION OF THE DRAWINGS

Non-limiting embodiments of the present disclosure are described by way of example with reference to the accompanying drawings, which are schematic and not intended to be drawn to scale. The accompanying drawings are provided for purposes of illustration only, and the dimensions, positions, order, and relative sizes reflected in the figures in the drawings may vary. For example, devices may be enlarged so that detail is discernable, but is intended to be scaled down in relation to, *e.g.,* fit within a working channel of a delivery catheter or endoscope. In the figures, identical or nearly identical or equivalent elements are typically represented by the same reference characters, and similar elements are typically designated with similar reference numbers differing in increments of 100, with redundant description omitted. For purposes of clarity and simplicity, not every element is labeled in every figure, nor is every element of each embodiment shown where illustration is not necessary to allow those of ordinary skill in the art to understand the disclosure.

The Detailed Description will be better understood in conjunction with the accompanying drawings, wherein like reference characters represent like elements, as follows:
**FIG. 1** depicts components of an endoscope;
**FIG. 2** depicts components of an endoscope system;
**FIGS. 3A** and **FIG. 3B** illustrates various features of an embodiment of an enclosure system according to the present disclosure;
**FIG. 4A** illustrates various features of an embodiment of an enclosure system according to the present disclosure;
**FIG. 4B** illustrates various features of installation of the enclosure system of **FIG. 4A** on a fluid container;
**FIGS. 5A** and **5B** illustrates various features of an enclosure system according to one or more embodiments of the present disclosure;
**FIGS. 6A** and **6B** illustrate various features of an embodiment of an enclosure system according to the present disclosure;
**FIGS. 6C-6E** illustrate various features of an embodiment of an enclosure system according to the present disclosure; and
**FIG. 7A-7C** illustrates various features of an enclosure system according to one or more embodiments of the present disclosure.

### DETAILED DESCRIPTION

The following Detailed Description should be read with reference to the drawings, which depict illustrative embodiments. It is to be understood that the present disclosure is not limited to the particular embodiments described, as such may vary. All apparatuses and systems and methods discussed herein are examples of apparatuses and/or systems and/or methods implemented in accordance with one or more principles of the present disclosure. Each example of an embodiment is provided by way of explanation and is not the only way to implement these principles but are merely examples. Thus, references to elements or structures or features in the drawings must be appreciated as references to examples of embodiments of the present disclosure, and should not be understood as limiting the present disclosure to the specific elements, structures, or features illustrated. Other examples of manners of implementing the disclosed principles will occur to a person of ordinary skill in the art upon reading the present disclosure. It will be apparent to those skilled in the art that various modifications and variations can be made in the present disclosure without departing from the scope of the present subject matter. For instance, features illustrated or described as part of one embodiment can be used with another embodiment to yield a still further embodiment. Thus, it is intended that the present subject matter covers such modifications and variations as come within the scope of the appended claims and their equivalents.

The described technologies are generally directed to fluid container enclosures configured to be coupled to a fluid container, for example, to seal the fluid container while enabling access to the contents of the fluid container, such as from an endoscopic system via a tubing set. A fluid container may be or may include a bottle or reservoir (see for example, reservoir 270). The fluid container enclosures may be configured as a cap, lid, or other enclosure component capable of sealing a fluid container. In some embodiments, the fluid container enclosures may include openings, lumens, holes, or other elements capable of allowing tubing access to the contents of the fluid container while maintaining a seal. Fluid container enclosures according to some embodiments are configured to operate with fluid containers of various properties, such as size, neck configuration, thread configuration, and/or the like. Accordingly, fluid container enclosures according to some embodiments may operate as universal enclosures that are able to be used with a wide array of fluid container configurations, including fluid contains made by different manufacturers and fluid containers having different shapes, sizes, neck configurations, and/or the like.

Some challenges in coupling with a fluid container and gaining access to the contents of the fluid container may include having a fluid container enclosure that is compatible with the fluid container. For example, a fluid container enclosure may include a screw cap with one or more tubes extending therethrough. In such examples, the screw cap may couple to corresponding threads on a neck of the fluid container with the one or more tubes extending therethrough enabling the endoscopic system to access the contents of the fluid container. However, there are many different types of fluid container manufacturers that offer different fluid container designs. Further, manufacturers may offer different fluid container designs and/or periodically change or update fluid container designs. For instance, manufacturers may offer designs with different thread patterns or neck sizes around the world based on regional preferences or demands. This presents a challenge for manufacturers of tubing sets by requiring them to offer multiple products with customized fluid container enclosures for each design. Further, product acquisition and stocking by health care facilities is complicated by necessitating that they ensure that tubing sets have a fluid container enclosure that is compatible with an available fluid container.

Accordingly, various embodiments of the present disclosure include fluid container enclosures that widen the scope of compatibility to a variety of different fluid container designs. In many embodiments, one or more fluid container enclosures of the present disclosure may provide an efficient, safe, and effective way to couple with and gain access to the contents of a multitude of fluid container designs. Enabling fluid container enclosures to be compatible with different fluid container designs allows manufacturers of tubing sets to offer products that are more adaptable and appeal to a broader market. Further, enabling fluid container enclosures to be compatible with different fluid container designs can simplify product acquisition and stocking by health care facilities.

For example, enclosure systems according to some embodiments may be used with a wide variety of fluid container opening or neck dimensions, such as an opening diameter and/or thread configuration (for instance, Glass Packaging Institute (GPI) thread finish or "H" dimension). In some embodiments, an enclosure system may include a cap capable of being used to seal a fluid container having an opening size of about 0.5 centimeters (cm), about 1 cm, about 2 cm, about 3 cm, about 4 cm, about 5 cm, about 10 cm, about 20 cm, and any value or range between any two of these values (including endpoints). In some embodiments, an enclosure system may include a cap capable of being used to seal a fluid container having a GPI thread finish of 350, 400, 410, 415, 425, 430, 450, and any value or range between any two of these values (including endpoints). The described embodiments may provide additional advantages that would be known to those of skill in the art.

It may be understood that the disclosure included herein is exemplary and explanatory only and is not restrictive. As used herein, the terms "comprises," "comprising," or any other variation thereof, are intended to cover a non-exclusive inclusion, such that a process, method, article, or apparatus that comprises a list of elements does not include only those elements, but may include other elements not expressly listed or inherent to such process, method, article, or apparatus. The term "exemplary" is used in the sense of "example," rather than "ideal." Although endoscopes and endoscopic systems are referenced herein, reference to endoscopes, endoscopic systems, or endoscopy should not be construed as limiting the possible applications of the disclosed features. For example, the disclosed features may be used in conjunction with duodenoscopes, bronchoscopes, ureteroscopes, colonoscopes, catheters, diagnostic or therapeutic tools or devices, or other types of medical devices or systems.

Reference is now made to the drawings, wherein like reference numerals are used to refer to like elements throughout. In the following description, for purpose of explanation, numerous specific details are set forth in order to provide a thorough understanding thereof. It may be evident, however, that the novel embodiments can be practiced without these specific details. In other instances, well known structures and devices are shown in block diagram form to facilitate a description thereof. The intention is to cover all modification, equivalents, and alternatives within the scope of the claims.

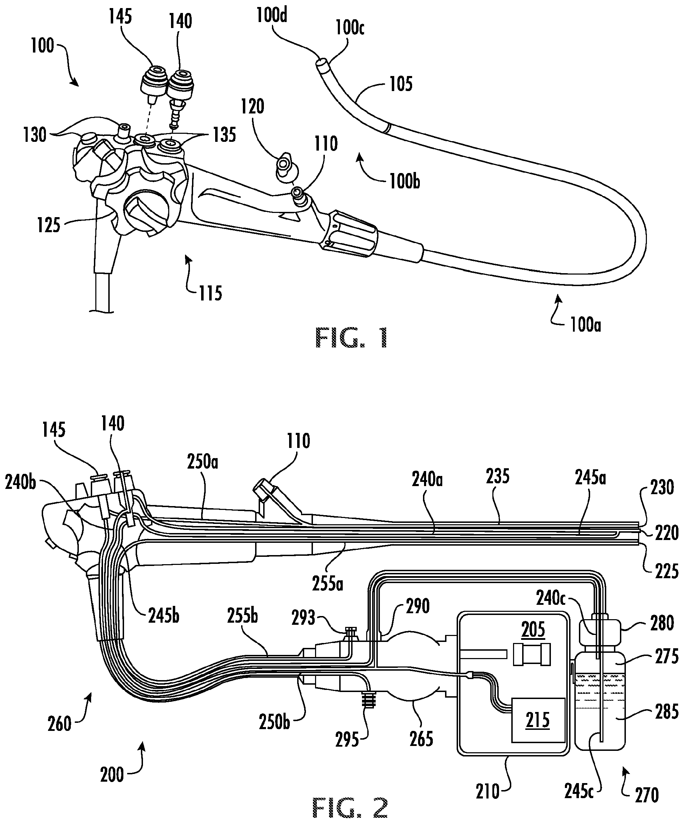

With reference to **FIGS. 1-2****,** an exemplary endoscope 100 and system 200 is depicted that may comprise an elongated shaft 100a that is inserted into a patient. A light source 205 feeds illumination light to a distal portion 100b of the endoscope 100, which may house an imager (e.g., CCD or CMOS imager) (not shown). The light source 205 (e.g., lamp) is housed in a video processing unit 210 that processes signals that are input from the imager and outputs processed video signals to a video monitor (not shown) for viewing. The video processing unit 210 also serves as a component of an air/water feed circuit by housing a pressurizing pump 215, such as an air feed pump, in the unit.

The endoscope shaft 100a may include a distal tip 100c provided at the distal portion 100b of the shaft 100a and a flexible bending portion 105 proximal to the distal tip 100c. The flexible bending portion 105 may include an articulation joint (not shown) to assist with steering the distal tip 100c. On an end face 100d of the distal tip of the endoscope 100 is a gas/lens wash nozzle 220 for supplying gas to insufflate the interior of the patient at the treatment area and for supplying water to wash a lens covering the imager. An irrigation opening 225 in the end face 100d supplies irrigation fluid to the treatment area of the patient. Illumination windows (not shown) that convey illumination light to the treatment area, and an opening 230 to a working channel 235 extending along the shaft 100a for passing tools to the treatment area, also may be included on the face 100d of the distal tip 100c. The working channel 235 extends along the shaft 100a to a proximal channel opening 110 positioned distal to an operating handle 115 of the endoscope 100. A biopsy valve 120 may be utilized to seal the channel opening 110 against unwanted fluid egress.

The operating handle 115 may be provided with knobs 125 for providing remote 4-way steering of the distal tip via wires connected to the articulation joint in the bendable flexible portion 105 (e.g., one knob controls up-down steering and another knob control for left-right steering). A plurality of video switches 130 for remotely operating the video processing unit 210 may be arranged on a proximal end side of the handle 115. In addition, the handle is provided with dual valve wells 135 that receive a gas/water valve 140 for operating an insufflating gas and lens water feed operation. A gas supply line 240a and a lens wash supply line 245a run distally from the gas/water valve 140 along the shaft 100a and converge at the distal tip 100c proximal to the gas/wash nozzle 220 (FIG. 2). The other valve well 135 receives a suction valve 145 for operating a suction operation. A suction supply line 250a runs distally from the suction valve 145 along the shaft 100a to a junction point in fluid communication with the working channel 235 of the endoscope 100.

The operating handle 115 is electrically and fluidly connected to the video processing unit 210, via a flexible umbilical 260 and connector portion 265 extending therebetween. The flexible umbilical 260 has a gas (e.g., air or CO2) feed line 240b, a lens wash feed line 245b, a suction feed line 250b, an irrigation feed line 255b, a light guide (not shown), and an electrical signal cable. The connector portion 265 when plugged into the video processing unit 210 connects the light source 205 in the video processing unit with the light guide. The light guide runs along the umbilical 260 and the length of the endoscope shaft 100a to transmit light to the distal tip 100c of the endoscope 100. The connector portion 265 when plugged into the video processing unit 210 also connects the air pump 215 to the gas feed line 240b in the umbilical 260.

A water reservoir 270 (e.g., water bottle) is fluidly connected to the endoscope 100 through the connector portion 265 and the umbilical 260. A length of gas supply tubing 240c passes from one end positioned in an air gap 275 between the top 280 (e.g., bottle cap or enclosure) of the reservoir 270 and the remaining water 285 in the reservoir to a detachable gas/lens wash connection 290 on the outside of the connector portion 265. The gas feed line 240b from the umbilical 260 branches in the connector portion 265 to fluidly communicate with the gas supply tubing 240c at the detachable gas/lens wash connection 290, as well as the air pump 215. A length of lens wash tubing 245c, with one end positioned at the bottom of the reservoir 270, passes through the top (e.g., a cap or enclosure) 280 of the reservoir 270 to the same detachable connection 290 as the gas supply tubing 240c on the connector portion 265. In other embodiments, the connections may be separate and/or separated from each other. The connector portion 265 also has a detachable irrigation connection 293 for irrigation supply tubing (not shown) running from a source of irrigation water (not shown) to the irrigation feed line 255b in the umbilical 260. In some embodiments, irrigation water is supplied via a pump (e.g., peristaltic pump) from a water source independent (not shown) from the water reservoir 270. In other embodiments, the irrigation supply tubing and lens wash tubing 245c may source water from the same reservoir. The connector portion 265 may also include a detachable suction connection 291 for suction feed line 250b and suction supply line 250a fluidly connecting a vacuum source (e.g., hospital house suction) (not shown) to the umbilical 260 and endoscope 100.

The gas feed line 240b and lens wash feed line 245b are fluidly connected to the valve well 135 for the gas/water valve 140 and configured such that operation of the gas/water valve in the well controls supply of gas or lens wash to the distal tip 100c of the endoscope 100. The suction feed line 250b is fluidly connected to the valve well 135 for the suction valve 145 and configured such that operation of the suction valve in the well controls suction applied to the working channel 235 of the endoscope 100.

Referring to FIG. 2, an exemplary operation of an endoscopic system 200, including an endoscope such as endoscope 100 above, is explained. Air from the air pump 215 in the video processing unit 210 is flowed through the connection portion 265 and branched to the gas/water valve 140 on the operating handle 115 through the gas feed line 240b in the umbilical 260, as well as through the gas supply tubing 240c to the water reservoir 270 via the connection 290 on the connector portion 265. When the gas/water valve 140 is in a neutral position, without the user's finger on the valve, air is allowed to flow out of the valve to atmosphere. In a first position, the user's finger is used to block the vent to atmosphere. Gas is allowed to flow from the valve 140 down the gas supply line 240a and out the distal tip 100c of the endoscope 100 in order to, for example, insufflate the treatment area of the patient. When the gas/water valve 140 is pressed downward to a second position, gas is blocked from exiting the valve, allowing pressure of the air passing from the air pump 215 to rise in the water reservoir 270. Pressurizing the water source forces water out of the lens wash tubing 245c, through the connector portion 265, umbilical 260, through the gas/water valve 140 and down the lens wash supply line 245a, converging with the gas supply line 240a prior to exiting the distal tip 100c of the endoscope 100 via the gas/lens wash nozzle 220. Air pump pressure may be calibrated to provide lens wash water at a relatively low flow rate compared to the supply of irrigation water.

The volume of the flow rate of the lens wash is governed by gas pressure in the water reservoir 270. When gas pressure begins to drop in the water reservoir 270, as water is pushed out of the reservoir 270 through the lens wash tubing 245c, the air pump 215 replaces lost air supply in the reservoir 270 to maintain a substantially constant pressure, which in turn provides for a substantially constant lens wash flow rate. In some embodiments, a filter (not shown) may be placed in the path of the gas supply tubing 240c to filter-out undesired contaminants or particulates from passing into the water reservoir 270. In some embodiments, outflow check valves or other 1-way valve configurations (not shown) may be placed in the path of the lens wash supply tubing to help prevent water from back-flowing into the reservoir 270 after the water has passed the valve.

A relatively higher flow rate compared to lens wash is typically required for irrigation water, since a primary use is to clear the treatment area in the patient of debris that obstructs the user's field of view. Irrigation is typically achieved with the use of a pump (e.g., peristaltic pump), as described. In embodiments with an independent water source for irrigation, tubing placed in the bottom of a water source is passed through the top of the water source and threaded through the head on the upstream side of the pump. Tubing on the downstream side of the pump 255c is connected to the irrigation feed line 255b in the umbilical 260 and the irrigation supply line 255a endoscope 100 via the irrigation connection 293 on the connector portion 265. When irrigation water is required, fluid is pumped from the water source by operating the irrigation pump, such as by depressing a footswitch (not shown), and flows through the irrigation connection 293, through the irrigation feed line 255b in the umbilical, and down the irrigation supply line in the shaft 100a of the endoscope to the distal tip 100c. In order to equalize the pressure in the water source as water is pumped out of the irrigation supply tubing, an air vent (not shown) may be included in the top (e.g., a cap or enclosure) 280 of the water reservoir 270. The vent allows atmospheric air into the water source preventing negative pressure build-up in the water source, which could create a vacuum that suctions undesired matter from the patient back through the endoscope toward the water source. In some embodiments, outflow check valves or other 1-way valve configurations (not shown), similar to the lens wash tubing 245c, may be placed in the path of the irrigation supply tubing to help prevent back-flow into the reservoir after water has passed the valve.

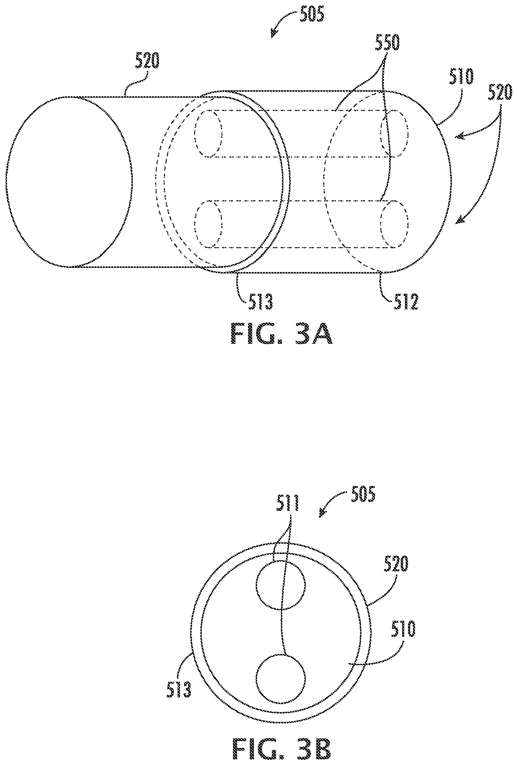

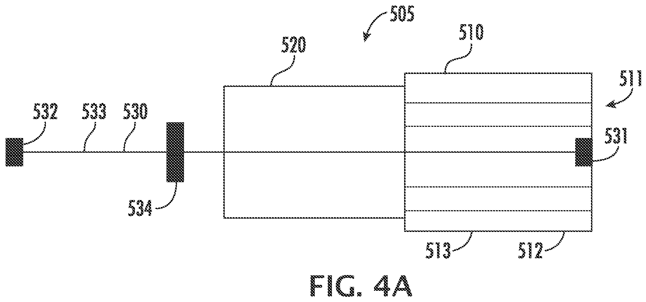

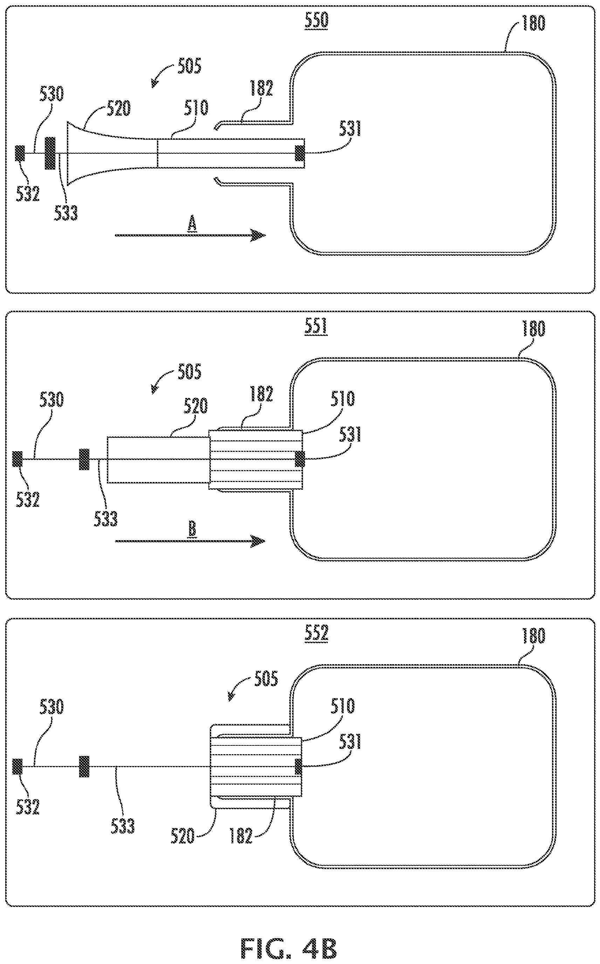

**FIGS. 3A** and **FIG. 3B** illustrates various features of an embodiment of an enclosure system according to the present disclosure. In particular, FIG. 3A depicts a side perspective view and FIG. 3B depicts a top view of a plug-based enclosure system 505 having a plug 510 with a first (inserted or anterior) end 512 arranged to be inserted into a fluid container (see, for example, FIGS. 4A and 4B) and a second (non-inserted or posterior) end 513 having a flexible sleeve 520 extending therefrom. In some embodiments, sleeve 520 may be hollow, for example, formed the same or similar to a flexible cylinder.

In various embodiments, enclosure system 505 may be formed as a single piece. For example, plug 510 may be formed of a first material (for instance, rigid silicone) that transitions into sleeve 520 (for instance, a more flexible form of silicone). In other embodiments, enclosure system 505 may be formed of separate plug 510 and sleeve 520 portions coupled together, for example, via an adhesive, fastener, or other coupling component.

In various embodiments, plug 510 may be formed of a rigid yet deformable material. In some embodiments, plug 510 may be formed of a polymer, an elastomer, silicone, rubber, cork, a wax, variations thereof, and/or combinations thereof. In various embodiments, plug 510 may be formed of silicone. In some embodiments, plug 510 may be formed of a rigid form of silicone, for example, with a durometer scale of between about 55A to about 100A. Plug 510 may be deformed such that its diameter (for example, outer diameter) decreases in order to fit within a neck (or other inlet/outlet port) of a fluid container, such as a bottle.

In exemplary embodiments, one or more holes or openings 511 may be formed in plug 510 to accommodate, for instance, tubing 550 configured to extend into the fluid container to access the contents thereof. In some embodiments, openings 511 may include or may be associated with a liner or insert configured to maintain the size and/or shape of openings 511 when plug 510 is being deformed to fit within a neck of a fluid container. For example, a rigid material, including, without limitation, silicone or a polymer, may be arranged within at least a portion of openings 511 to prevent or reduce the deformation of openings 511. Accordingly, tubes 550 extending through openings 511 may not be closed off due to the deformation of plug 510, allowing the flow of fluid and/or gas from/to the fluid container.

In various embodiments, sleeve 520 may be formed of a material capable of allowing sleeve 520 to be deformed to fold down over the neck of the fluid container after plug 510 has been installed (see, for example, state 552 of FIG. 4B). In this manner, sleeve 520 may operate to provide or enhance the sealing properties of enclosure system 505. In some embodiments, sleeve may be formed of a polymer, an elastomer, silicone, rubber, variations thereof, and/or combinations thereof. In various embodiments, sleeve 520 may be formed of silicone. In some embodiments, sleeve 520 may be formed of a highly-flexible form of silicone, for example, silicone on a low-durometer scale (for instance, between about 5OO to about 50A).

In one embodiment, to install enclosure system 505 in a fluid container, a user may apply pressure to a portion of plug 510, such as by squeezing plug 510 at or near first end 512, to decrease a diameter of plug 510 to fit into the neck of the fluid container. The user may move first end 512 into the neck of the fluid container and remove the pressure to allow the diameter of plug 510 at the deformed portion to expand to create a seal against the inner walls or surfaces of the neck of the fluid container. Enclosure system 505 may be further secured to the fluid container by rolling sleeve 520 over at least a portion of the neck of the fluid container. In some embodiments, the sizing and/or flexibility of sleeve 520 may facilitate sleeve 520 tightly conforming and/or locking with any threads on an outside surface of the neck of the fluid container (for instance, regardless of the particular thread size, shape, or other configuration).

FIG. 4A illustrates various features of an embodiment of an enclosure system according to the present disclosure. As shown in FIG. 4A, plug-based enclosure system 505 may include a plunger 530 having a plunger rod 533 extending axially through plug 510 and a stem 534 for engaging fingers of a user when pressing down on plunger rod 533. An anchor 531 may be arranged on first end 512 of plug 510 to affix plunger rod 533 to plug 510. For example, movement of plunger rod 533 may push or pull on first end 512 of plug. In FIG. 4A, enclosure system 505 is depicted in an initial inactive state prior to installation in a fluid container.

**FIG. 4B** illustrates various features of installation of the enclosure system of FIG. 4A on a fluid container. As shown in step 550, applying a force to plunger 530, for instance, by pushing on handle 532 (e.g., using a thumb of a user similar to using a plunger of a syringe to expel fluid from the syringe), to move plunger rod 533 in direction A while maintaining plug 510 in the same or substantially the same position (for instance, through an opposing force by fingers engaging stem 534) may cause plug 510 (and sleeve 520) to deform, for example, to stretch and become longer and decrease an outer diameter of plug 510. Once deformed by forcing plunger 530 to a sufficient diameter, plug 510 may be moved into a neck 182 of a fluid container 180 (for example, a bottle). Referring to step 551, once plug 510 is positioned as desired within neck 182, the user may release the force on plunger 530, causing plunger rod 533 to move in direction B (for example, as deformed plug 510 attempts to move back to its original, relaxed shape). The outer diameter of plug 510 expands, and an outer surface of plug 510 may press against an inner surface of neck 182 to affix plug 510 within neck 182. In some embodiments, plug 510 may form a water-tight, gas-tight, hermetic, or other type of seal with neck 182.

Referring to step 552, once plug 510 has expanded within neck 182 and created a seal, the user can roll sleeve 520 over the outer surface of neck 204, for example, to provide for additional sealing and/or holding of enclosure system 505 and fluid container 180. In addition, enclosure system may allow a user to secure enclosure system 505 via plunger 530 using one hand (for instance, allowing the user to steady fluid container 180 bottle with their other hand if desired).

In some embodiments, once installed, plunger 530 or plunger rod 533 may be removed from plug 510. For example, plunger rod 533 may be threaded to mate with anchor 531. In another example, plunger rod 533 may form snap-fit, friction fit, or other type of removable physical connection with anchor 531. To remove an installed plug 510, plunger rod 533 may be re-connected with anchor 531 and then pushed in direction A to reduce an outer diameter of plug.

Although a plunger 530 is used in the illustrative example depicted in FIG. 4B, embodiments are not so limited. For instance, enclosure system 505 may use any type of component capable of deforming plug 510 to fit within neck 182, for example, a releasable and/or removable clamp, vise, sleeve, and/or the like. In another instance, enclosure system 505 may not use a separate component to deform plug, instead, for instance, relying on manual pressure by a user to deform and/or push plug 510 into neck to an adequate position.

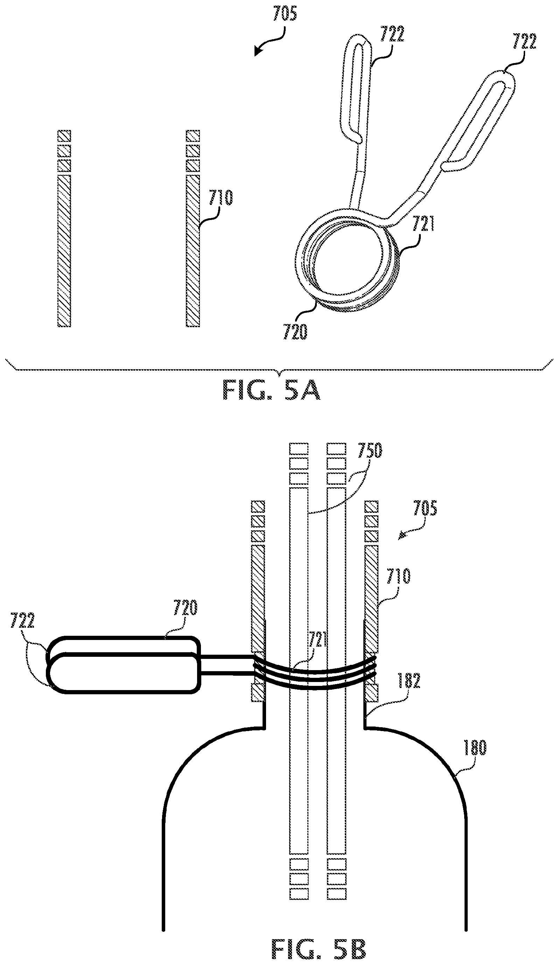

**FIGS. 5A** and **5B** illustrates an embodiment of an enclosure system according to one or more embodiments of the present disclosure. As shown in FIG. 5A, a clamp-based enclosure system 705 may include an enclosure 710 and a clamp 720. In some embodiments, enclosure 710 may be or may include a tube or tube portion. In various embodiments, enclosure 710 may be formed of a flexible material, such as a polymer, silicone, an elastomer, polyvinyl chloride (PVC), a polyolefin, polyethylene, polyurethane, variations thereof, and/or combinations thereof. In various embodiments, clamp 720 may include a spring-based clamp or clip having handles 722 and a clamp (spring) portion 721. In general, squeezing handles 722 together may cause clamp portion 721 to open (for instance, increase an inner diameter of the spring), releasing handles 722 may cause clamp portion to close (for instance, decrease the inner diameter of the spring). In general, clamp 720 may be biased toward the closed position.

FIG. 5B depicts enclosure system 705 installed on a neck 182 of a fluid container 100. In some embodiments, the original cap or other enclosure that comes with bottle would be removed and replaced with enclosure 710 configured to be fed over at least a portion of neck 182. Inside of the tube formed by enclosure 710, tubing 750 may extend into fluid container 180 to allow tubing 750 to access the contents of fluid container 180. In some embodiments, enclosure 710 may be sized to form a friction fit with neck 182. In addition, clamp 720 may be arranged around a portion of enclosure 710 covering neck to secure or further secure enclosure 710 to neck 182 and/or to form or enhance a seal between enclosure 710 and neck 182.

To install enclosure system 705 on fluid container 180 as depicted in FIG. 5B, enclosure 710 may be forced or otherwise positioned around neck 182. Clamp 720 may be initially positioned around neck 182 in an open configuration and enclosure 710 passed down through enclosure, or clamp may be passed down along enclosure 710 from a top portion (e.g., distal from neck 182) to the bottom portion of enclosure 710 that will be positioned around neck 182. Once enclosure 710 is positioned around neck 182, the opening force on clamp 720 may be released to cause clamp portion 721 to clamp around enclosure 710 and neck 182.

Although a spring clamp is used in the example depicted in FIGS. 5A and 5B, embodiments are not so limited. For instance, various other clamp or clamp-like components may be used instead of or in combination with claim 720, such as a hose clamp, a collar, a barbell collar-like mechanism, a clip, and/or the like.

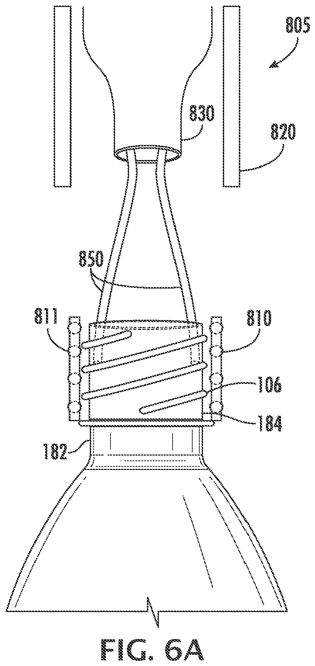

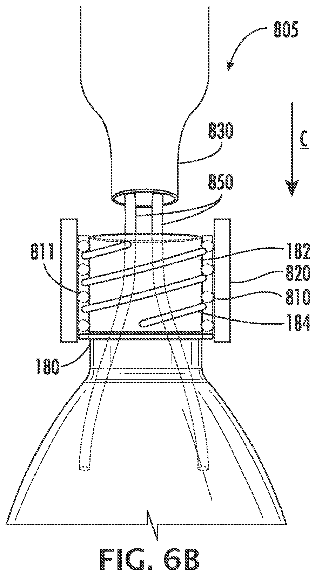

**FIGS. 6A** and **6B** illustrate an embodiment of an enclosure system according to the present disclosure. As shown in FIGS. 6A and 6B, a ball bearing (or quick disconnect) based enclosure system 805 may include a set of ball bearings 811 arranged within an enclosure 810, such as a flexible tube. For example, ball bearings 811 may be arranged within a wall of a tube forming enclosure 810. The enclosure 810 may be fed over a neck 182 of a fluid container 180, with ball bearings 811 floating freely (or relatively freely) over threads 184. Once enclosure 810 is in position over neck 182, a sleeve 820 may be forced over enclosure 810, forcing ball bearings 811 into a position that compresses them against neck 182. In some embodiments, ball bearings 811 may be spaced so that ball bearings are arranged between threads 184. In various embodiments, enclosure 810 may be formed of a flexible material, such as a polymer, silicone, an elastomer, polyvinyl chloride (PVC), a polyolefin, polyethylene, polyurethane, variations thereof, and/or combinations thereof.

In various embodiments, sleeve 820 may be biased to move in direction C, for example, by a spring or other biasing element (not shown). Accordingly, enclosure 810 may be pushed onto neck 182 while sleeve 820 is being held in position above enclosure. Once enclosure 810 is in proper position about neck 182, sleeve 820 may be released, causing sleeve 820 to move in direction C, over enclosure 810 portion around neck 182. In this manner, enclosure system 805 may have portions that act the same or similar to a quick connect coupling, for instance, sleeve 820 may operate the same or similar as a spring-biased female coupling component of a quick connect coupling.

In some embodiments, tubing 850 may extend into fluid container 180 through enclosure system 805 to allow tubing 850 to access the contents of fluid container 180. For example, enclosure system 805 may include a conduit 830 for providing a passage for tubing 850 to extend into fluid container 180.

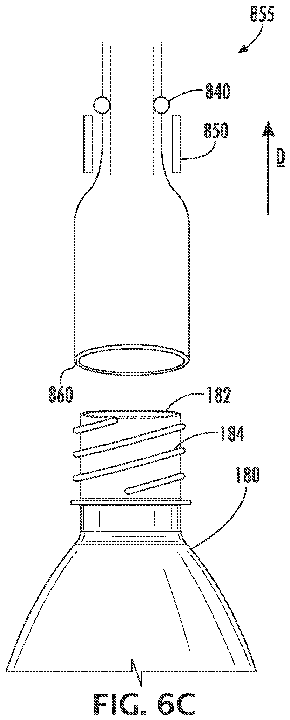

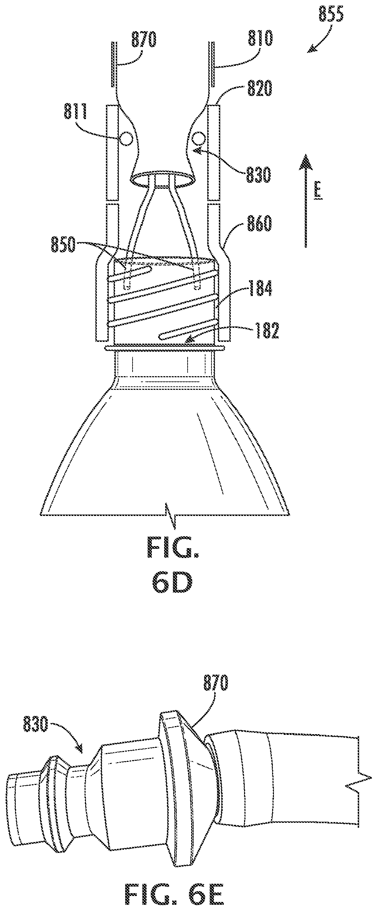

**FIGS. 6C-6E** illustrate an embodiment of an enclosure system according to the present disclosure. As shown in FIGS. 6C and 6D, a ball bearing-based enclosure system 855 may include an enclosure 860 configured to be positioned about neck 182 of fluid container 180. Enclosure 860 may be formed of a flexible material, including, without limitation, a polymer, silicone, rubber, and combinations thereof. In various embodiments, enclosure 860 may form a friction fit with neck 182. In some embodiments, enclosure system 855 may have a sleeve 850 biased in direction D, for instance, by a spring or other biasing element. While enclosure 860 is being positioned about neck 182 by a user, sleeve 850 may be held in a downward position toward neck 182 against the biasing force.

Referring to FIG. 6D, when enclosure 860 is suitably positioned about neck 182, sleeve 820 may be released, causing sleeve to move in direction E. In some embodiments, sleeve 820 may move a specified distance, for example, due to a stop or a limitation of the biasing force. When sleeve 820 slides up in direction E, an inner surface of sleeve 820 may engage ball bearings 811 and force ball bearings to be seated in a groove 830 or other structure of a mating element 870. In some embodiments, mating element 870 may be the same or similar to a male quick connect or fast-action coupler element, for example, as depicted in FIG. 6E. Tubing 850 may extend through mating element 870 into container 180.

When enclosure system 805 is in the installed state, enclosure 860 may form a seal with neck 182 alone or in combination with tube 810 and/or mating element 870. Engagement of mating element 870 via ball bearings 811 may operate to maintain the coupling between enclosure system 855 and fluid container 802. Pushing down on sleeve 820 (for example, in a direction toward fluid container 180) may allow ball bearings 811 to unseat from groove 830 to allow for release of enclosure system 855 from fluid container 180.

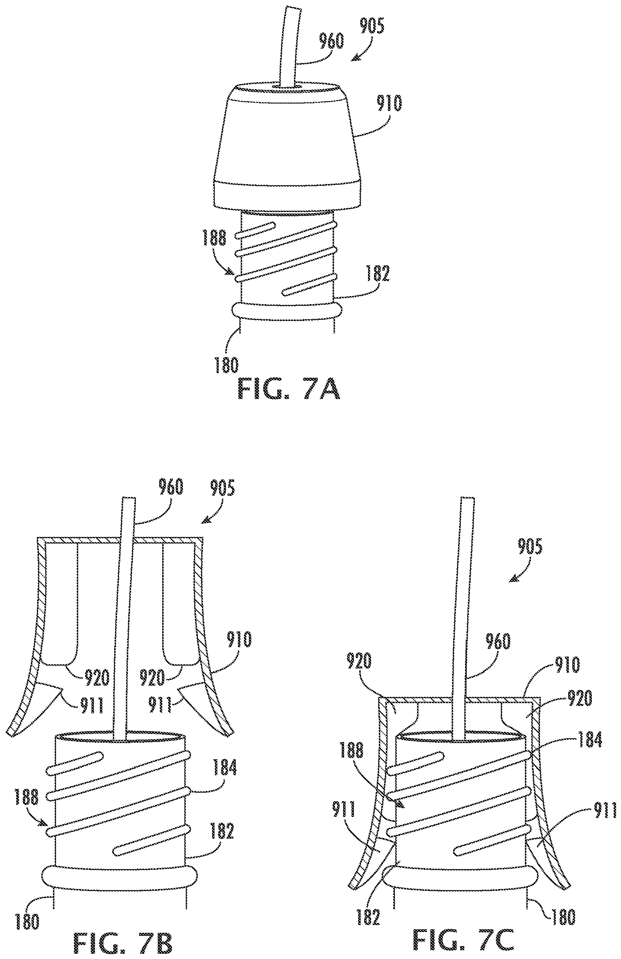

**FIG. 7A-7C** illustrates an embodiment of an enclosure system according to one or more embodiments of the present disclosure. FIG. 7A shows an external view of a sheath-based enclosure system 905 that includes an enclosure, hood, or cap 910 configured to be arranged about a neck 182 of a fluid container 180. In some embodiments, cap 910 may have a conical or substantially conical shape. In various embodiments, cap 910 may be biased to a given inner diameter by an external semiflexible sheath that will give under force while maintaining a constant radial pressure. Cap 910 may be configured to have one or more tubes 960 extend therethrough to access contents of fluid container 180.

FIG. 7B depicts a sectional view of enclosure system 905 showing cap in an uninstalled state. As shown in FIG. 7B, cap 910 may include internal features, such as protrusions or a ridge 911 extending from an internal bottom or base portion of cap. For example, in some embodiments, protrusions 911 may include one or more hooks, teeth, or other elements extending from an internal surface of cap 910. In various embodiments, protrusions 911 may be configured to catch or otherwise engage distended features on the external surface of neck 182, such as threads 184.

In some embodiments, cap 910 and/or protrusions 911 may be formed of a flexible material, such as a polymer, rubber, silicone, and/or the like. In various embodiments, protrusions could be any material that is acceptable from a biocompatibility perspective while maintaining the ability to adhere to the features on neck 182 of fluid container 180. In some embodiments, protrusions 911 may extend circumferentially or substantially circumferentially around an inner portion of cap, for instance, forming a ridge, boss, or shoulder. In various embodiments, a ridge, a protrusion, or a portion of protrusions 911 may be formed at a base or bottom portion of cap 910. In other embodiments, a ridge, a protrusion, or a portion of protrusions 911 may be formed further up the inner side wall or surface of cap 910.

In various embodiments, a sealing material 920 may be arranged on an underside of cap 910, for example, on upper top and/or side surfaces of cap 910. In some embodiments, sealing material 920 may be formed of various malleable materials, such as a polymer, silicone, (malleable) wax, variations thereof, combinations thereof, and/or the like. In various embodiments, sealing material 920 may be formed of a low durometer polymer or silicone, such as a material with a durometer scale of about 5A to about 50A. In some embodiments, sealing material 920 may have a stickiness or tackiness characteristic. In exemplary embodiments, sealing material 920 may circumferentially fill a top portion of cap 910 to create a seal when cap 910 is pressed onto neck 182 (see, for example, FIG. 7C).

Referring to FIG. 7C, to install cap 910 on neck 182, cap 910 may be pressed down onto neck 182 and/or ratcheted around neck 182. The flexible material of cap 910 may allow protrusions 911 to expand outward from neck 182 to allow cap 910 to be pressed downward onto neck 182. For example, cap 910 may be pushed down on neck 182, expanding out beyond threads 184. Because protrusions 911 are biased toward neck 182, protrusions 911 may become seated within grooves between threads 184 when in the area of grooves 188. In some embodiments, the flexible material of cap 910 and shape of protrusions 911 (for instance, a hook or hook-like shape) may allow biased protrusions 911 to ride or ratchet around grooves formed between threads 184 such that cap 910 may be rotated onto necks 104 of various shapes, dimensions, and other physical characteristics by riding through the grooves between threads 184 to become seated as depicted in FIG. 7C. Cap 910 may be pressed down and/or threaded down around neck to allow sealing material 920 to form a seal with neck. In some embodiments, cap 910 may be installed on neck 182 to maintain a certain pressure, for example, a pressure required by fluid container 180 during operation (for instance, about 8 psi to move fluid out of fluid container 180).

Once cap 910 is installed, movement of cap 910 away from fluid container 180 may be prevented due to engagement of protrusions 911 with threads 102. In addition, sealing material 920 may act the same or similar to an adhesive, particularly a weak, temporary adhesive, to affix cap 910 to neck 182 and form a water-tight, gas-tight, or hermetic seal between cap 910 and neck 182.

The foregoing discussion has broad application and has been presented for purposes of illustration and description and is not intended to limit the disclosure to the form or forms disclosed herein. It will be understood that various additions, modifications, and substitutions may be made to embodiments disclosed herein without departing from the scope of the present disclosure. In particular, it will be clear to those skilled in the art that principles of the present disclosure may be embodied in other forms, structures, arrangements, proportions, and with other elements, materials, and components, without departing from the scope, or characteristics thereof. For example, various features of the disclosure are grouped together in one or more features, embodiments, or configurations for the purpose of streamlining the disclosure. However, it should be understood that various features of the certain features, embodiments, or configurations of the disclosure may be combined in alternate features, embodiments, or configurations. While the disclosure is presented in terms of embodiments, it should be appreciated that the various separate features of the present subject matter need not all be present in order to achieve at least some of the desired characteristics and / or benefits of the present subject matter or such individual features. One skilled in the art will appreciate that the disclosure may be used with many modifications or modifications of structure, arrangement, proportions, materials, components, and otherwise, used in the practice of the disclosure, which are particularly adapted to specific environments and operative requirements without departing from the scope of the present disclosure. For example, elements shown as integrally formed may be constructed of multiple parts or elements shown as multiple parts may be integrally formed, the operation of elements may be reversed or otherwise varied, the size or dimensions of the elements may be varied. Similarly, while operations or actions or procedures are described in a particular order, this should not be understood as requiring such particular order, or that all operations or actions or procedures are to be performed, to achieve desirable results. Additionally, other implementations are within the scope of the following claims. In some cases, the actions recited in the claims can be performed in a different order and still achieve desirable results. The presently disclosed embodiments are therefore to be considered in all respects as illustrative and not restrictive, the scope of the claimed subject matter being indicated by the appended claims, and not limited to the foregoing description or particular embodiments or arrangements described or illustrated herein. In view of the foregoing, individual features of any embodiment may be used and can be claimed separately or in combination with features of that embodiment or any other embodiment, the scope of the subject matter being indicated by the appended claims, and not limited to the foregoing description.

In the foregoing description and the following claims, the following will be appreciated. The phrases "at least one", "one or more", and "and/or", as used herein, are openended expressions that are both conjunctive and disjunctive in operation. The terms "a", "an", "the", "first", "second", etc., do not preclude a plurality. For example, the term "a" or "an" entity, as used herein, refers to one or more of that entity. As such, the terms "a" (or "an"), "one or more" and "at least one" can be used interchangeably herein. All directional references (e.g., proximal, distal, upper, lower, upward, downward, left, right, lateral, longitudinal, front, back, top, bottom, above, below, vertical, horizontal, radial, axial, clockwise, counterclockwise, and/or the like) are only used for identification purposes to aid the reader's understanding of the present disclosure, and/or serve to distinguish regions of the associated elements from one another, and do not limit the associated element, particularly as to the position, orientation, or use of this disclosure. Connection references (e.g., attached, coupled, connected, and joined) are to be construed broadly and may include intermediate members between a collection of elements and relative movement between elements unless otherwise indicated. As such, connection references do not necessarily infer that two elements are directly connected and in fixed relation to each other. Identification references (e.g., primary, secondary, first, second, third, fourth, etc.) are not intended to connote importance or priority, but are used to distinguish one feature from another. The following claims are hereby incorporated into this Detailed Description by this reference, with each claim standing on its own as a separate embodiment of the present disclosure. Reference signs in the claims are provided merely as a clarifying example and shall not be construed as limiting the scope of the claims in any way.

The following claims are hereby incorporated into this Detailed Description by this reference, with each claim standing on its own as a separate embodiment of the present disclosure. In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "the", "first", "second", etc., do not preclude a plurality. Reference signs in the claims are provided merely as a clarifying example and shall not be construed as limiting the scope of the claims in any way.

## Claims

1. An enclosure system (505) for enclosing a fluid container (180), comprising:
a plug (510) having an outer diameter larger than an opening of a neck of the fluid container, a first plug end (512) and a second plug end (513) arranged on opposing sides of the plug, the plug configured to deform responsive to pressure to reduce the outer diameter to fit the first plug end through the opening and into the neck (182) of the fluid container (180); and
a sleeve (520) forming a hollow flexible cylinder extending from the second plug end (513), the sleeve having a first sleeve end coupled to the second plug end (513) and a second sleeve end arranged opposite the first sleeve end, the first sleeve end configured to fold at least a portion of the sleeve (520) outward and down toward the second plug end (513) to arrange at least a portion of the sleeve (520) over an outer neck surface of the neck (182) when the first plug end (512) is installed within the neck (182).

2. The enclosure system (505) according to claim 1, the first plug end (512) to expand to engage an inner surface of the neck (182) to form a seal responsive to removal of the pressure.

3. The enclosure system (505) according to one of claims 1 or 2, an outer sleeve surface of the sleeve to engage the outer neck surface when the sleeve (520) is folded over the outer neck surface to form a seal between the sleeve (520) and the neck (182).

4. The enclosure system (505) according to any one of the preceding claims, wherein at least one opening (511) is formed in the second plug end (513) for at least one tube of a tube set to extend through the plug to access a fluid arranged with the fluid container (180).

5. The enclosure system (505) according to claim 4, further comprising at least one liner arranged within the at least one opening (511) to maintain a shape of opening when the plug (520) is deformed.

6. The enclosure system (505) according to any one of the preceding claims, further comprising a plunger (530) having a plunger rod (533) extending through the sleeve (520) and the plug (510), the plunger rod having a first plunger end anchored to the first plug end and a second plunger end arranged opposite the first plunger end, the second plunger end having a plunger handle (532).

7. The enclosure system (505) according to claim 6, the plunger handle (532) configured to move in a direction toward the first plug end (513) responsive to a force to cause the first plunger end to pull on the first plug end (513) to stretch the plug and reduce the outer diameter to fit the first end in the opening of the neck (182).

8. The enclosure system (505) according to claim 7, the first plug end (513) to expand to engage an inner surface of the neck to form a seal responsive to removal of the force.

9. The enclosure system (505) according to any of claims 6-8, the plunger rod (533) removably coupled to an anchor (531).

## Patentansprüche

1. Verschluss-System (505) zum Verschließen eines Fluidbehälters (180), aufweisend:
einen Stopfen (510) mit einem Außendurchmesser, der größer als eine Öffnung eines Halses des Fluidbehälters ist, einem ersten Stopfenende (512) und einem zweiten Stopfenende (513), die auf gegenüberliegenden Seiten des Stopfens angeordnet sind, wobei der Stopfen so konfiguriert ist, dass er sich als Reaktion auf Druck verformt, um den Außendurchmesser zu verringern, damit das erste Stopfenende durch die Öffnung und in den Hals (182) des Fluidbehälters (180) passt; und
eine Hülse (520), die einen hohlen flexiblen Zylinder bildet, der sich von dem zweiten Stopfenende (513) aus erstreckt, wobei die Hülse ein erstes Hülsenende, das mit dem zweiten Stopfenende (513) verbunden ist, und ein zweites Hülsenende, das dem ersten Hülsenende gegenüberliegend angeordnet ist, aufweist, wobei das erste Hülsenende so konfiguriert ist, dass es mindestens einen Abschnitt der Hülse (520) nach außen und nach unten zum zweiten Stopfenende (513) hin faltet, um mindestens einen Abschnitt der Hülse (520) über einer äußeren Halsfläche des Halses (182) anzuordnen, wenn das erste Stopfenende (512) in dem Hals (182) installiert ist.

2. Verschluss-System (505) nach Anspruch 1, wobei sich das erste Stopfenende (512) ausdehnt, um eine Innenfläche des Halses (182) in Eingriff zu nehmen und eine Dichtung zu bilden, die auf Wegfall des Drucks reagiert.

3. Verschluss-System (505) nach einem der Ansprüche 1 oder 2, wobei eine äußere Hülsenfläche der Hülse die äußere Halsfläche in Eingriff nimmt, wenn die Hülse (520) über die äußere Halsfläche gefaltet wird, um eine Dichtung zwischen der Hülse (520) und dem Hals (182) zu bilden.

4. Verschluss-System (505) nach einem der vorstehenden Ansprüche, wobei mindestens eine Öffnung (511) in dem zweiten Stopfenende (513) für mindestens einen Schlauch eines Schlauchsets ausgebildet ist, der sich durch den Stopfen erstreckt, um Zugang zu einem mit dem Fluidbehälter (180) angeordneten Fluid zu erhalten.

5. Verschluss-System (505) nach Anspruch 4, ferner aufweisend mindestens eine Auskleidung, die in der mindestens einen Öffnung (511) angeordnet ist, um eine Form der Öffnung beizubehalten, wenn der Stopfen (520) verformt wird.

6. Verschluss-System (505) nach einem der vorstehenden Ansprüche, ferner aufweisend einen Kolben (530) mit einer Kolbenstange (533), die sich durch die Hülse (520) und den Stopfen (510) erstreckt, wobei die Kolbenstange ein erstes Kolbenende, das an dem ersten Stopfenende verankert ist, und ein zweites Kolbenende, das gegenüber dem ersten Kolbenende angeordnet ist, aufweist, wobei das zweite Kolbenende einen Kolbengriff (532) aufweist.

7. Verschluss-System (505) nach Anspruch 6, wobei der Kolbengriff (532) so konfiguriert ist, dass er sich in eine Richtung zum ersten Stopfenende (513) hin bewegt und auf eine Kraft reagiert, die bewirkt, dass das erste Kolbenende am ersten Stopfenende (513) zieht, um den Stopfen zu strecken und den Außendurchmesser zu verringern, damit das erste Ende in die Öffnung des Halses (182) passt.

8. Verschluss-System (505) nach Anspruch 7, wobei sich das erste Stopfenende (513) ausdehnt, um eine Innenfläche des Halses in Eingriff zu nehmen und eine Dichtung zu bilden, die auf den Wegfall der Kraft reagiert.

9. Verschluss-System (505) nach einem der Ansprüche 6-8, wobei die Kolbenstange (533) lösbar mit einem Anker (531) verbunden ist.

## Revendications

1. Système de fermeture (505) pour fermer un récipient de fluide (180), comprenant :
un bouchon (510) ayant un diamètre externe plus grand qu'une ouverture d'un goulot du récipient de fluide, une première extrémité de bouchon (512) et une deuxième extrémité de bouchon (513) agencées sur les côtés opposés du bouchon, le bouchon étant configuré pour se déformer en réponse à la pression afin de réduire le diamètre externe pour adapter la première extrémité de bouchon à travers l'ouverture et dans le goulot (182) du récipient de fluide (180) ; et
un manchon (520) formant un cylindre flexible creux s'étendant à partir de la deuxième extrémité de bouchon (513), le manchon ayant une première extrémité de manchon couplée à la deuxième extrémité de bouchon (513) et une deuxième extrémité de manchon agencée à l'opposé de la première extrémité de manchon, la première extrémité de manchon étant configurée pour plier au moins une partie du manchon (520) vers l'extérieur et vers le bas vers la deuxième extrémité de bouchon (513) pour agencer au moins une partie du manchon (520) sur une surface de goulot externe du goulot (182) lorsque la première extrémité de bouchon (512) est installée à l'intérieur du goulot (182).

2. Système de fermeture (505) selon la revendication 1, la première extrémité de bouchon (512) étant prévue pour subir une expansion afin de mettre en prise une surface interne du goulot (182) pour former un joint d'étanchéité sensible à la suppression de la pression.

3. Système de fermeture (505) selon l'une des revendications 1 ou 2, une surface de manchon externe du manchon étant prévue pour mettre en prise la surface de goulot externe lorsque le manchon (520) est plié sur la surface de goulot externe afin de former un joint d'étanchéité entre le manchon (520) et le goulot (182).

4. Système de fermeture (505) selon l'une quelconque des revendications précédentes, dans lequel au moins une ouverture (511) est formée dans la deuxième extrémité de bouchon (513) pour au moins un tube d'un ensemble de tubes afin de s'étendre à travers le bouchon pour avoir accès à un fluide agencé avec le récipient de fluide (180).

5. Système de fermeture (505) selon la revendication 4, comprenant en outre au moins un revêtement agencé à l'intérieur de la au moins une ouverture (511) afin de maintenir une forme d'ouverture lorsque le bouchon (520) est déformé.

6. Système de fermeture (505) selon l'une quelconque des revendications précédentes, comprenant en outre un piston plongeur (530) ayant une tige de piston plongeur (533) s'étendant à travers le manchon (520) et le bouchon (510), la tige de piston plongeur ayant une première extrémité de piston plongeur ancrée sur la première extrémité de bouchon et une deuxième extrémité de piston plongeur agencée à l'opposé de la première extrémité de piston plongeur, la deuxième extrémité de piston plongeur ayant une poignée de piston plongeur (532).

7. Système de fermeture (505) selon la revendication 6, la poignée de piston plongeur (532) étant configurée pour se déplacer dans une direction vers la première extrémité de bouchon (513) en réponse à une force pour amener la première extrémité de piston plongeur à tirer sur la première extrémité de piston plongeur (513) afin d'étirer le bouchon et réduire le diamètre externe pour adapter la première extrémité dans l'ouverture du goulot (182).

8. Système de fermeture (505) selon la revendication 7, la première extrémité de bouchon (513) étant prévue pour subir une expansion afin de mettre en prise une surface interne du goulot pour former un joint d'étanchéité sensible à la suppression de la force.

9. Système de fermeture (505) selon l'une quelconque des revendications 6 à 8, la tige de piston plongeur (533) étant couplée, de manière amovible, à un ancrage (531).
